# EUROPÄISCHE PATENTSCHRIFT

(11) **EP 1 324 978 B1**
(45) Veröffentlichungstag und Bekanntmachung des Hinweises auf die Patenterteilung: **22.03.2006**
(21) Anmeldenummer: 01969768.9
(22) Anmeldetag: 25.09.2001
(51) Int. Cl.: C07C 253/34, C07C 255/07

(54) **VERFAHREN ZUR TRENNUNG VON PENTENNITRIL-ISOMEREN**
METHOD FOR SEPARATING PENTENENITRILE ISOMERS
PROCEDE DE SEPARATION D'ISOMERES DE PENTENENITRILE

(30) Priorität: 28.09.2000 DE 10049265
(43) Veröffentlichungstag der Anmeldung: 09.07.2003
(73) Patentinhaber: BASF Aktiengesellschaft, 67056 Ludwigshafen (DE)
(72) Erfinder: JUNGKAMP, Tim, 69207 Sandhausen (DE); KUNSMANN-KEITEL, Dagmar, Pascale, 67117 Limburgerhof (DE); BAUMANN, Robert, 68161 Mannheim (DE); BASSLER, Peter, 68519 Viernheim (DE); SIEGEL, Wolfgang, 67117 Limburgerhof (DE)
(86) Internationale Anmeldenummer: PCT/EP2001/011050
(87) Internationale Veröffentlichungsnummer: WO 2002/026698

(56) Entgegenhaltungen:
- US-A- 3 852 327
- US-A- 3 865 865

## Beschreibung

Die vorliegende Erfindung betrifft ein Verfahren zur destillativen Auftrennung von Pentennitril-Isomeren, die im Druckbereich von 1 bis 500 kPa eine relative Flüchtigkeit alpha im Bereich von 1,0 bis 1,3 aufweisen, dadurch gekennzeichnet, daß man die Destillation in Gegenwart eines flüssigen Verdünnungsmittels durchführt, das mit den Pentennitril-Isomeren unter gleichen Druckbedingungen Azeotrope bildet, deren relative Flüchtigkeit alpha höher ist als die der zu trennenden Pentennitril-Isomere.

Zur großtechnischen Herstellung von Polyamiden besteht weltweit ein großer Bedarf an alpha,omega-Alkylendiaminen, welche dabei als ein wichtiges Ausgangsprodukt dienen. Alpha,omega-Alkylendiamine, wie z.B. das Hexamethylendiamin, werden fast ausschließlich durch Hydrierung der entsprechenden Dinitrile gewonnen. Fast alle großtechnischen Wege zur Herstellung von Hexamethylendiamin sind daher im wesentlichen Varianten der Herstellung des Adipodinitrils, von dem jährlich weltweit etwa 1,0 Mio. Tonnen produziert werden.

In K. Weissermel, H.-J. Arpe, Industrielle Organische Chemie, 4. Auflage, VCH Weinheim, S. 266 ff. sind vier prinzipiell unterschiedliche Routen zur Herstellung von Adipinsäuredinitril beschrieben:
1. die dehydratisierende Aminierung der Adipinsäure mit Ammoniak in der Flüssig- oder Gasphase über intermediär gebildetes Diamid;
2. die indirekte Hydrocyanierung des 1,3-Butadiens über die Zwischenstufe der 1,4-Dichlorbutene;
3. die Hydrodimerisierung von Acrylnitril in einem elektrochemischen Prozess; und
4. die direkte Hydrocyanierung von 1,3-Butadien mit Cyanwasserstoff.

Nach dem letztgenannten Verfahren erhält man in einer ersten Stufe durch Monoaddition ein Gemisch isomerer Pentennitrile, wie trans-2-Pentennitril, cis-2-Pentennitril, trans-3-Pentennitril, cis-3-Pentennitril, 4-Pentennitril, (E)-2-Methyl-2-butennitril, (Z)-2-Methyl-2-butennitril und 2-Methyl-3-butennitril.

Anschließend wird in einer weiteren Stufe durch anti-Markownikow-Cyanwasserstoffaddition an 4-Pentennitril das Adipinsäuredinitril gebildet. Die Umsetzung erfolgt dabei in der Flüssigphase in einem Lösungsmittel, wie z. B. Tetrahydrofuran, bei einer Temperatur im Bereich von 30 - 150 °C und drucklos. Dabei werden als Katalysatoren Nickelkomplexe mit Phosphor-haltigen Liganden und gegebenenfalls Lewis-Säuren wie Metallsalze oder Triphenlybor als Promotoren verwendet. Auch bei dieser zweiten Cyanwasserstoffaddition werden aus 4-Pentennitril oder den damit im Gleichgewicht vorliegenden Isomeren, wie trans-3-Pentennitril, cis-3-Pentennitril und 2-Methyl-3-butennitril oder deren Gemischen, andere Pentennitril-Isomere gebildet.

In "Applied Homogeneous Catalysis with Organometalic Compounds", Bd. 1, VCH Weinheim, S. 465 ff. wird allgemein die heterogen und homogen katalysierte Addition von Cyanwasserstoff an Olefine beschrieben. Dabei werden vor allem Katalysatoren auf Basis von Phosphin-, Phosphit- und Phosphinit-Komplexen des Nickels und Palladiums verwendet. Zur Herstellung von Adipinsäuredinitril durch Hydrocyanierung von Butadien werden vorwiegend Nickel(0)-Phosphitkatalysatoren, ggf. in Gegenwart einer LewisSäure als Promotor verwendet. Bei der Bildung des Monoadditionsproduktes erhält man ein Isomerengemisch, welches u.a. 3-Pentennitril und 2-Methyl-3-butennitril umfasst.

Die WO 99/13983 beschreibt die Hydrocyanierung von Butadien oder eines 1,3-Butadien-haltigen Kohlenwasserstoffgemischs zu monoolefinischen C₅-Monanitrilen und/oder Adipodinitril in Gegenwart von Phosphonit-Komplexen des Nickels. Hierbei werden ebenfalls Isoemerengemische von Pentennitrilen erhalten.

Vor der zweiten Cyanwasserstoffaddition ist eine Abtrennung von solchen Pentennitril-Isomeren erwünscht, die sich nur schlecht zu Adipinsäuredinitril umsetzen lassen oder Nebenprodukte bilden. Ebenso ist eine Auftrennung des Pentennitril-Isomeren-Gemisches erwünscht, das bei der Adipodinitrilsynthese als Nebenprodukt erhalten wird.

Die destillative Auftrennung dieses Isomeren-Gemisches bereitet erhebliche Probleme, da die relative Flüchtigkeit alpha bestimmter Pentennitril-Isomere im Bereich von 1 bis 500 kPa im Bereich von 1,0 bis 1,3 liegt. Unter der relativen Flüchtigkeit alpha versteht man dabei den Quotienten der Dampfdrücke zweier Substanzen, wobei man den Dampfdruck der Substanz mit dem höheren Dampfdruck in den Zähler des Quotienten nimmt.

Hierzu kommen insbesondere Mischungen in Betracht, die die Pentennitril-Kombinationen trans-3-Pentennitril / 4-Pentennitril, trans-3-Pentennitril / trans-2-Pentennitril, trans-2-Pentennitril / 4-Pentennitril, cis-3-Pentennitril / 4-Pentennitril, cis-3-Pentennitril / trans-2-Pentennitril oder (E)-2-Methyl-2-butennitril / 2-Methyl-3-butennitril enthalten.

Zu Umgehung des Trennproblems trans-3-Pentennitril / trans-2-Pentennitril wurde beispielsweise in US 3,526,654, US 3,564,040, US 3,852,325 und US 3,852,327 vorgeschlagen, die schlecht destillativ abtrennbaren Pentennitril-Isomere katalytisch in solche umzuwandeln, die sich leicht destillativ abtrennen lassen.

Nachteilig hierbei ist, daß die katalytische Isomerisierung durch Bildung von unerwünschten Isomeren oder Oligomeren zu Verlusten an Wertprodukt führt.

Der vorliegenden Erfindung lag die Aufgabe zugrunde, ein Verfahren bereitzustellen, das die destillative Auftrennung von Pentennitril-Isomeren, die im Druckbereich von 1 bis 500 kPa eine relative Flüchtigkeit alpha im Bereich von 1,0 bis 1,3 aufweisen, auf technisch einfache und wirtschaftliche Weise ermöglicht.

Demgemäß wurde das eingangs definierte Verfahren gefunden.

Das erfindungsgemäße Verfahren läßt sich vorteilhaft auf die Trennung von Pentennitril-Isomeren, die im Druckbereich von 1 bis 500 kPa eine relative Flüchtigkeit alpha im Bereich von 1,0 bis 1,3, vorzugsweise 1,0 bis 1,2, insbesondere 1,0 bis 1,15 aufweisen, anwenden. Hierbei kommen insbesondere Mischungen in Betracht, die die oben genannten Pentennitril-Isomeren-Kombinationen enthalten.

In einer bevorzugten Ausführungsform kommt ein Verfahren in Betracht, bei dem man aus einer Mischung, enthaltend trans-3-Pentennitril und trans-2-Pentennitril eine Mischung erhält, die ein höheres Verhältnis an trans-2-Pentennitril zu trans-3-Pentennitril aufweist als die Ausgangsmischung, und eine Mischung, die ein niedrigeres Verhältnis an trans-2-Pentennitril zu trans-3-Pentennitril aufweist als die Ausgangsmischung.

In einer weiteren bevorzugten Ausführungsform kommt ein Verfahren in Betracht, bei dem man aus einer Mischung, enthaltend trans-3-Pentennitril und 4-Pentennitril eine Mischung erhält, die ein höheres Verhältnis an trans-3-Pentennitril zu 4-Pentennitril aufweist als die Ausgangsmischung, und eine Mischung, die ein niedrigeres Verhältnis an trans-3-Pentennitril zu 4-Pentennitril aufweist als die Ausgangsmischung.

In einer weiteren bevorzugten Ausführungsform kommt ein Verfahren in Betracht, bei dem man aus einer Mischung, enthaltend trans-2-Pentennitril und 4-Pentennitril eine Mischung erhält, die ein höheres Verhältnis an trans-2-Pentennitril zu 4-Pentennitril aufweist als die Ausgangsmischung, und eine Mischung, die ein niedrigeres Verhältnis an trans-2-Pentennitril zu 4-Pentennitril aufweist als die Ausgangsmischung.

In einer weiteren bevorzugten Ausführungsform kommt ein Verfahren in Betracht, bei dem man aus einer Mischung, enthaltend cis-3-Pentennitril und 4-Pentennitril eine Mischung erhält, die ein höheres Verhältnis an cis-3-Pentennitril zu 4-Pentennitril aufweist als die Ausgangsmischung, und eine Mischung, die ein niedrigeres Verhältnis an cis-3-Pentennitril zu 4-Pentennitril aufweist als die Ausgangsmischung.

In einer weiteren bevorzugten Ausführungsform kommt ein Verfahren in Betracht, bei dem man aus einer Mischung, enthaltend cis-3-Pentennitril und trans-2-Pentennitril eine Mischung erhält, die ein höheres Verhältnis an cis-3-Pentennitril zu trans-2-Pentennitril aufweist als die Ausgangsmischung, und eine Mischung, die ein niedrigeres Verhältnis an cis-3-Pentennitril zu trans-2-Pentennitril aufweist als die Ausgangsmischung.

In einer weiteren bevorzugten Ausführungsform kommt ein Verfahren in Betracht, bei dem man aus einer Mischung, enthaltend (E)-2-Methyl-2-butennitril und 2-Methyl-3-butennitril eine Mischung erhält, die ein höheres Verhältnis an (E)-2-Methyl-2-butennitril zu 2-Methyl-3-butennitril aufweist als die Ausgangsmischung, und eine Mischung, die ein niedrigeres Verhältnis an (E)-2-Methyl-2-butennitril zu 2-Methyl-3-butennitril aufweist als die Ausgangsmischung.

Die Herstellung der im erfindungsgemäßen Verfahren eingesetzten Mischung von Pentennitril-Isomeren, insbesondere durch Cyanwasserstoffaddition an Butadien oder Butadien enthaltenden Kohlenwasserstoffgemischen, kann nach an sich bekannten Verfahren, insbesondere durch Cyanwasserstoffaddition an Butadien oder Butadien enthaltenden Kohlenwasserstoffgemischen, beispielsweise wie eingangs beschrieben erfolgen. Grundsätzlich wird nach bisherigen Beobachtungen die Anwendung des erfindungsgemäßen Verfahrens durch die Art der Herstellung der Pentennitril-Isomeren oder durch die mengenmäßige Zusammensetzung der Isomeren-Mischung nicht eingeschränkt oder ausgeschlossen.

Erfindungsgemäß führt man die Destillation in Gegenwart eines flüssigen Verdünnungsmittels durchführt, das mit den Pentennitril-Isomeren unter gleichen Druckbedingungen jeweils Azeotrope bildet, deren relative Flüchtigkeit alpha höher, vorzugsweise um mindestens 1 % höher, ist als die der zu trennenden Pentennitril-Isomere.

Die Menge an flüssigem Verdünnungsmittel gegenüber der Menge an Pentennitril-Isomeren-Gemisch ist an sich nicht kritisch. Setzt man mehr flüssiges Verdünnungsmittel ein als den durch die Azeotrope abzudestillierenden Mengen entspricht, so verbleibt überschüssiges flüssiges Verdünnungsmittel als Sumpfprodukt. Setzt man weniger flüssiges Verdünnungsmittel ein als den durch die Azeotrope abzudestillierenden Mengen entspricht, so verbleibt überschüssiges Pentennitril-Isomeren-Gemisch als Sumpfprodukt. Es hat sich als vorteilhaft erwiesen, das flüssige Verdünnungsmittel gegenüber dem Pentennitril-Isomeren-Gemisch in der Menge einzusetzen, die den durch die Azeotrope abzudestillierenden Mengen entspricht. Die Destillation kann man vorteilhaft bei einem Druck im Bereich von 1 bis 200 kPa, vorzugsweise 50 bis 100 kPa, insbesondere bei Umgebungsdruck durchführen.

Die Destillation kann vorteilhaft durch fraktionierende Destillation in einer oder mehreren, wie 2 oder 3 Destillationsapparaturen erfolgen.

Dabei kommen für die Destillation hierfür übliche Apparaturen in Betracht, wie sie beispielsweise in: Kirk-Othmer, Encyclopedia of Chemical Technology, 3. Ed., vol. 7, John Wiley & Sons, New York, 1979, Seite 870-881 beschrieben sind, wie Siebbodenkolonnen, Glockenbodenkolonnen, Packungskolonnen, Füllkörperkolonnen, Kolonnen mit Seitenabzug oder Trennwandkolonnen.

In einer bevorzugten Ausführungsform setzt man ein flüssiges Verdünnungsmittel ein, das mit Pentennitril-Isomeren eine Mischungslücke unter bestimmten Mengenbedingungen, Druckbedingungen, vorzugsweise im Bereich von 10 bis 200 kPa, insbesondere unter Normaldruck, und Temperaturbedingungen, vorzugsweise im Bereich von 0 bis 120°C, insbesondere bei Umgebungstemperatur, aufweist.

Vorzugsweise kann eine Auftrennung der Produktmischung in zwei Phasen durch Wahl einer geeigneten Temperatur erreicht werden. Als weitere Möglichkeit kommt die Wahl geeigneter Mengenverhältnisse in Betracht wie der Zusatz von flüssigem Verdünnungsmittel.

Die Phasentrennung kann in an sich bekannter Weise in für solche Zwecke beschriebenen Apparaturen erfolgen, wie sie beispielsweise aus: Ullmann's Encyclopedia of Industrial Chemistry, Vol. B3, 5. Ed., VCH Verlagsgesellschaft, Weinheim, 1988, Seite 6-14 bis 6-22 bekannt sind.

Die für die Phasentrennung optimalen Apparaturen und Verfahrensbedingungen lassen sich dabei leicht durch einige einfache Vorversuche ermitteln.

Vorteilhaft kann diejenige der beiden Phasen, die nach der Phasentrennung den höheren Gewichtsanteil an flüssigem Verdünnungsmittel aufweist, teilweise oder vollständig in das erfindungsgemäße Verfahren zurückgeführt werden.

Als flüssiges Verdünnungsmittel kommen organische oder anorganische flüssige Verdünnungsmittel in Betracht. In einer vorteilhaften Ausführungsform kann man Wasser als flüssiges Verdünnungsmittel einsetzten.

Die bei dem erfindungsgemäßen Verfahren erhaltenen Pentennitril-Isomere, die einer weiteren wirtschaftlichen Cyanwasserstoffaddition zugeführt werden können, können anschließend in an sich bekannter Weise zu Adipinsäuredinitril umgesetzt werden.

Die bei dem erfindungsgemäßen Verfahren erhaltenen Pentennitril-Isomere, die nicht einer weiteren wirtschaftlichen Cyanwasserstoffaddition zugeführt werden können, können anschließend in an sich bekannter Weise zu anderen Pentennitril-Isomeren isomerisiert werden.

### Beispiele

### Erfindungsgemäße Beispiele

Eine Mischung aus 800 g eines Pentennitril-Isomeren-Gemisches gemäß Tabelle 1 und 800 g Wasser würde in einer Destillationskolonne (Höhe 185 cm, Durchmesser 30 mm, Packung: Sulzerpack EX, Firma Sulzer Chemtec AG, Winterthur, Schweiz) bei einem Rücklaufverhältnis von 1:5 (Abnahme:Rücklauf) unter Umgebungsdruck fraktionierend destilliert. Die als Kopffraktion erhaltenen Proben wurden gaschromatographisch analysiert.

### Vergleichsbeispiele

Die Vergleichsbeispiele wurden wie die erfindungsgemäßen Beispiele durchgeführt, jedoch wurden 1600 g Pentennitril-Isomeren-Gemisch gemäß Tabelle 1 eingesetzt und kein Wasser.

**Tabelle 1**

| | Pentennitril-Isomeren-Kombination | GC-Flächenverhältnis | | Destill.-Temp. |
|---|---|---|---|---|
| | | Vor Destill. | Nach Destill. | |
| Bsp. 1 | trans-2-Pentennitril / trans-3-Pentennitril | 1,9 | 3,4 | 92°C |
| Vgl.1 | trans-2-Pentennitril/trans-3-Pentennitril | 2,2 | 1,6 | 142°C |
| Bsp. 2 | trans-3-Pentennitril / 4-Pentennitril | 4,5 | 26 | 94°C |
| Vgl.2 | trans-3-Pentennitril/4-Pentennitril | 4,4 | 7,1 | 132°C |
| Bsp.3 | (E)-2-Methyl-2-butennitril/ 2-Methyl-3-butennitril | 6,0 | 20 | 87°C |
| Vgl.3 | (E)-2-Methyl-2-butennitril/ 2-Methyl-3-butennitril | 6,0 | 14 | 117°C |

## Patentansprüche

1. Verfahren zur destillativen Auftrennung von Pentennitril-Isomeren, die im Druckbereich von 1 bis 500 kPa eine relative Flüchtigkeit alpha im Bereich von 1,0 bis 1,3 aufweisen, **dadurch gekennzeichnet, daß** man die Destillation in Gegenwart eines flüssigen Verdünnungsmittels durchführt, das mit den Pentennitril-Isomeren unter gleichen Druckbedingungen Azeotrope bildet, deren relative Flüchtigkeit alpha höher ist als die der zu trennenden Pentennitril-Isomere.

2. Verfahren nach Anspruch 1, wobei das flüssige Verdünnungsmittel mit Pentennitril-Isomeren eine Mischungslücke unter bestimmten Mengen-, Druck- und Temperaturbedingungen aufweist.

3. Verfahren nach Anspruch 1 oder 2, wobei man als flüssiges Verdünnungsmittel Wasser einsetzt.

4. Verfahren nach Anspruch 2 oder 3, wobei man die bei der Destillation erhaltene Mischung in die Mischungslücke überführt und diejenige der beiden Phasen, die den höheren Gewichtsanteil an flüssigem Verdünnungsmittel aufweist, in das Verfahren gemäß Anspruch 1 zurückführt.

5. Verfahren nach den Ansprüchen 1 bis 4, wobei man aus einer Mischung, enthaltend trans-3-Pentennitril und trans-2-Pentennitril eine Mischung erhält, die ein höheres Verhältnis an trans-2-Pentennitril zu trans-3-Pentennitril aufweist als die Ausgangsmischung, und eine Mischung, die ein niedrigeres Verhältnis an trans-2-Pentennitril zu trans-3-Pentennitril aufweist als die Ausgangsmischung.

6. Verfahren nach den Ansprüchen 1 bis 4, wobei man aus einer Mischung, enthaltend trans-3-Pentennitril und 4-Pentennitril eine Mischung erhält, die ein höheres Verhältnis an trans-3-Pentennitril zu 4-Pentennitril aufweist als die Ausgangsmischung, und eine Mischung, die ein niedrigeres Verhältnis an trans-3-Pentennitril zu 4-Pentennitril aufweist als die Ausgangsmischung.

7. Verfahren nach den Ansprüchen 1 bis 4, wobei man aus einer Mischung, enthaltend trans-2-Pentennitril und 4-Pentennitril eine Mischung erhält, die ein höheres Verhältnis an trans-2-Pentennitril zu 4-Pentennitril aufweist als die Ausgangsmischung, und eine Mischung, die ein niedrigeres Verhältnis an trans-2-Pentennitril zu 4-Pentennitril aufweist als die Ausgangsmischung.

8. Verfahren nach den Ansprüchen 1 bis 4, wobei man aus einer Mischung, enthaltend cis-3-Pentennitril und 4-Pentennitril eine Mischung erhält, die ein höheres Verhältnis an cis-3-Pentennitril zu 4-Pentennitril aufweist als die Ausgangsmischung, und eine Mischung, die ein niedrigeres Verhältnis an cis-3-Pentennitril zu 4-Pentennitril aufweist als die Ausgangsmischung.

9. Verfahren nach den Ansprüchen 1 bis 4, wobei man aus einer Mischung, enthaltend cis-3-Pentennitril und trans-2-Pentennitril eine Mischung erhält, die ein höheres Verhältnis an cis-3-Pentennitril zu trans-2-Pentennitril aufweist als die Ausgangsmischung, und eine Mischung, die ein niedrigeres Verhältnis an cis-3-Pentennitril zu trans-2-Pentennitril aufweist als die Ausgangsmischung.

10. Verfahren nach den Ansprüchen 1 bis 4, wobei man aus einer Mischung, enthaltend (E)-2-Methyl-2-butennitril und 2-Methyl-3-butennitril eine Mischung erhält, die ein höheres Verhältnis an (E)-2-Methyl-2-butennitril zu 2-Methyl-3-butennitril aufweist als die Ausgangsmischung, und eine Mischung, die ein niedrigeres Verhältnis an (E)-2-Methyl-2-butennitril zu 2-Methyl-3-butennitril aufweist als die Ausgangsmischung.

## Claims

1. A process for the distillative separation of pentene nitrile isomers which have a relative volatility alpha ranging from 1.0 to 1.3 in the pressure range from 1 to 500 kPa, wherein the distillation is carried out in the presence of a liquid diluent which forms with the pentene nitrile isomers, under the same pressure conditions, azeotropes whose relative volatility alpha is higher than that of the pentene nitrile isomers to be separated.

2. The process according to claim 1 wherein the liquid diluent exhibits a miscibility gap with pentene nitrile isomers under specific quantity, pressure and temperature conditions.

3. The process according to claim 1 or 2 wherein the liquid diluent used is water.

4. The process according to claim 2 or 3 wherein the mixture obtained in the distillation is transferred to the miscibility gap and that phase of the two which contains the higher proportion by weight of liquid diluent is recycled into the process according to claim 1.

5. The process according to any of claims 1 to 4 wherein a mixture comprising trans-3-pentene nitrile and trans-2-pentene nitrile yields a mixture in which the ratio of trans-2-pentene nitrile to trans-3-pentene nitrile is higher than in the starting mixture, and a mixture in which the ratio of trans-2-pentene nitrile to trans-3-pentene nitrile is lower than in the starting mixture.

6. The process according to any of claims 1 to 4 wherein a mixture comprising trans-3-pentene nitrile and 4-pentene nitrile yields a mixture in which the ratio of trans-3-pentene nitrile to 4-pentene nitrile is higher than in the starting mixture, and a mixture in which the ratio of trans-3-pentene nitrile to 4-pentene nitrile is lower than in the starting mixture.

7. The process according to any of claims 1 to 4 wherein a mixture comprising trans-2-pentene nitrile and 4-pentene nitrile yields a mixture in which the ratio of trans-2-pentene nitrile to 4-pentene nitrile is higher than in the starting mixture, and a mixture in which the ratio of trans-2-pentene nitrile to 4-pentene nitrile is lower than in the starting mixture.

8. The process according to any of claims 1 to 4 wherein a mixture comprising cis-3-pentene nitrile and 4-pentene nitrile yields a mixture in which the ratio of cis-3-pentene nitrile to 4-pentene nitrile is higher than in the starting mixture, and a mixture in which the ratio of cis-3-pentene nitrile to 4-pentene nitrile is lower than in the starting mixture.

9. The process according to any of claims 1 to 4 wherein a mixture comprising cis-3-pentene nitrile and trans-2-pentene nitrile yields a mixture in which the ratio of cis-3-pentene nitrile to trans-2-pentene nitrile is higher than in the starting mixture, and a mixture in which the ratio of cis-3-pentene nitrile to trans-2-pentene nitrile is lower than in the starting mixture.

10. The process according to any of claims 1 to 4 wherein a mixture comprising (E)-2-methyl-2-butene nitrile and 2-methyl-3-butene nitrile yields a mixture in which the ratio of (E)-2-methyl-2-butene nitrile to 2-methyl-3-butene nitrile is higher than in the starting mixture, and a mixture in which the ratio of (E)-2-methyl-2-butene nitrile to 2-methyl-3-butene nitrile is lower than in the starting mixture.

## Revendications

1. Procédé de séparation par distillation d'isomères de pentènenitriles qui présentent dans la gamme de pression de 1 à 500 kPa une volatilité relative alpha de l'ordre de 1,0 à 1,3, **caractérisé en ce qu'**on effectue la distillation en présence d'un diluant liquide qui forme avec les isomères de pentènenitrile dans les mêmes conditions de pression des azéotropes dont la volatilité relative alpha est supérieure à celle des isomères de pentènenitrile à séparer.

2. Procédé selon la revendication 1, dans lequel le diluant liquide présente avec des isomères de pentènenitrile une lacune de miscibilité dans certaines conditions de quantité, de pression et de température.

3. Procédé selon la revendication 1 ou 2, dans lequel on utilise l'eau comme diluant liquide.

4. Procédé selon la revendication 2 ou 3, dans lequel on transfère le mélange obtenu lors de la distillation dans la lacune de miscibilité et on réinjecte celle des deux phases qui présente la part en poids en diluant liquide la plus élevée dans le procédé selon la revendication 1.

5. Procédé selon l'une des revendications 1 à 4, dans lequel on obtient à partir d'un mélange contenant du trans-3-pentènenitrile et du trans-2-pentènenitrile un mélange qui présente une proportion en trans-2-pentènenitrile par rapport au trans-3-pentènenitrile supérieure au mélange de départ et un mélange qui présente une proportion en trans-2-pentènenitrile par rapport au trans-3-pentènenitrile inférieure au mélange de départ.

6. Procédé selon l'une des revendications 1 à 4, dans lequel on obtient à partir d'un mélange contenant du trans-3-pentènenitrile et du 4-pentènenitrile un mélange qui présente une proportion en trans-3-pentènenitrile par rapport au 4-pentènenitrile supérieure au mélange de départ et un mélange qui présente une proportion en trans-3-pentènenitrile par rapport au 4-pentènenitrile inférieure au mélange de départ.

7. Procédé selon l'une des revendications 1 à 4, dans lequel on obtient à partir d'un mélange contenant du trans-2-pentènenitrile et du 4-pentènenitrile un mélange qui présente une proportion en trans-2-pentènenitrile par rapport au 4-pentènenitrile supérieure au mélange de départ et un mélange qui présente une proportion en trans-2-pentènenitrile par rapport au 4-pentènenitrile inférieure au mélange de départ.

8. Procédé selon l'une des revendications 1 à 4, dans lequel on obtient à partir d'un mélange contenant du cis-3-pentènenitrile et du 4-pentènenitrile un mélange qui présente une proportion en cis-3-pentènenitrile par rapport au 4-pentènenitrile supérieure au mélange de départ et un mélange qui présente une proportion en cis-3-pentènenitrile par rapport au 4-pentènenitrile inférieure au mélange de départ.

9. Procédé selon l'une des revendications 1 à 4, dans lequel on obtient à partir d'un mélange contenant du cis-3-pentènenitrile et du trans-2-pentènenitrile un mélange qui présente une proportion en cis-3-pentènenitrile par rapport au trans-2-pentènenitrile supérieure au mélange de départ et un mélange qui présente une proportion en cis-3-pentènenitrile par rapport au trans-2-pentènenitrile inférieure au mélange de départ.

10. Procédé selon l'une des revendications 1 à 4, dans lequel on obtient à partir d'un mélange contenant du (E)-2-méthyl-2-butènenitrile et du 2-méthyl-3-butènenitrile un mélange qui présente une proportion en (E)-2-méthyl-2-butènenitrile par rapport au 2-méthyl-3-butènenitrile supérieure au mélange de départ et un mélange qui présente une proportion en (E)-2-méthyl-2-butènenitrile par rapport au 2-méthyl-3-butènenitrile inférieure au mélange de départ.
